# EUROPEAN PATENT SPECIFICATION

(11) **EP 2 004 161 B1**
(45) Date of publication and mention of the grant of the patent: **23.05.2012**
(21) Application number: 07727920.6
(22) Date of filing: 10.04.2007
(51) Int. Cl.: A61K 31/07, A61K 31/202, A61K 31/352, A61K 31/375, A61P 17/00

(54) **ORAL COMPOSITION COMPRISING DHA AND GENISTEIN FOR ENHANCING SKIN PROPERTIES**
ORALE ZUSAMMENSETZUNG MIT DHA UND GENISTEIN ZUR VERBESSERUNG DER HAUTEIGENSCHAFTEN
COMPOSITION ORALE COMPRENANT DE L'ADH ET DE LA GÉNISTÉINE POUR AMÉLIORER L'APPARANCE CUTANÉ

(30) Priority: 12.04.2006 EP 06252020; 12.02.2007 EP 07102128
(43) Date of publication of application: 24.12.2008
(73) Proprietor: Unilever PLC, London EC4Y 0DY (GB); Unilever N.V., 3013 AL Rotterdam (NL)
(72) Inventor: CASEY, John, Bedford Bedfordshire MK44 1LQ (GB); JENKINS, Gail, Bedford Bedfordshire MK44 1LQ (GB); WAINWRIGHT, Linda, Jane, Bedford Bedfordshire MK44 1LQ (GB)
(74) Representative: Acham, Nicholas Clive
(86) International application number: PCT/EP2007/053452
(87) International publication number: WO 2007/116052

(56) References cited:
- WO-A-2006/056293
- US-A1- 2004 082 523
- US-B1- 6 335 038
- RHODES LESLEY E ET AL: "Dietary fish-oil supplementation in humans reduces UVB-erythemal sensitivity but increases epidermal lipid peroxidation" JOURNAL OF INVESTIGATIVE DERMATOLOGY, vol. 103, no. 2, 1994, pages 151-154, XP009069139 ISSN: 0022-202X
- RHODES LESLEY E ET AL: "Dietary fish oil reduces basal and ultraviolet B-generated PGE-2 levels in skin and increases the threshold to provocation of polymorphic light eruption" JOURNAL OF INVESTIGATIVE DERMATOLOGY, vol. 105, no. 4, 1995, pages 532-535, XP009069138 ISSN: 0022-202X
- WEI HUACHEN ET AL: "Isoflavone genistein: Photoprotection and clinical implications in dermatology." JOURNAL OF NUTRITION, vol. 133, no. 11S-I, November 2003 (2003-11), pages 3811S-3819S, XP009068868 & INTERNATIONAL RESEARCH CONFERENCE ON FOOD, NUTRITION, AND CANCER; WASHINGTON DC, USA; JULY 17-18, 2003 ISSN: 0022-3166

## Description

The present invention relates to a composition for enhancing skin appearance and to the use of a combination of active compounds.

Improving the appearance and feel of human skin has received a great deal of research effort. However, the vast majority of commercially available products address this problem by acting on the exterior of the skin, the most common form being a topical skin cream. However, such topical applications have their limitations and deal primarily with the dead surface layers of the skin. It is known that certain ingredients can provide improvements in skin appearance and texture from being ingested. Such ingredients thus act from the interior of the skin and therefore can provide greater opportunities for improving the skin by accessing the living interior. Furthermore, such an effect may be perceived by the general public as being more potent or medical in nature than a topical application.

Dietary fish oil is known to convey significant protection against UVR-induced erythema upon ingestion.

Carotenoids such as lycopene and β-carotene have also been shown to give significant protection against UVR-induced erythema when induced orally.

Likewise, vitamins E & C when taken orally in combination have also been shown to provide protection against UVR-induced erythema.

US 6,589,535 (Johnson & Johnson) discloses a nutritional supplement which contains an oil rich in ω-3 and ω-6 fatty acids and a carotenoid in combination to combat the harmful effects of xenobiotics on the skin, in particular on the skin's immune system. However, this is limited to food supplements such as capsules or tablets and does not disclose how such materials may be delivered via a beverage or other food product. Blackcurrant seed oil is preferred as the source of the fatty acids, however this contains the less efficaceous ω-3 PUFA α-linolenic acid and is not as rich overall in ω-3 PUFA's as fish oil.

US2003/0082275 discloses a drinkable ω-3 preparation, which is storage stable. The drink disclosed contains a very high level of oil and consequently is unstable, forming a two-phase beverage upon storage. A drink having 4wt% oil, giving an ω-3 concentration of 1.6 wt% is exemplified. Egg yolk is used as an emulsifier which contains approximately 8wt% lecithin.

Our co-pending international application no PCT/EP2005/011658 relates to stable consumable emulsions.

WO 02/074308 describes a composition for the prevention of osteoporosis which comprises a combination of isoflavones and polyunsaturated fatty acids.

US 5,976,606 relates to a process for obtaining DHA-containing tofu or soybean milk drink. The aim is to avoid the undesirable taste and/or smell of fish oil.

EP-A-1340427 discloses acidic milks containing EPA and/or DHA. The document aims to provide formulations that are stable against oxidation and phase separation.

WO2006056293, a document which was filed before the date of filing of the European patent application but published after that date, discloses a consumable emulsion, comprising at least 50% water, 0.2 to 5 wt% of an oil comprising at least 12wt% of docosahexaenoic acid and eicosapentaenoic acid, an antioxidant, a flavouring, at least 0.01 wt% food-grade phospholipid emulsifier and from 0.01 to 0.5 wt% soy isoflavone material wherein the soy isoflavone is selected from genistein and daidzein

There remains a need for compositions that can provide beneficial anti-ageing effects on skin. In particular, there is a need for compositions that can achieve enhanced effects on skin.

The present invention is based on the surprising finding of a synergistic effect between two compounds in their effect on skin cells.

According to the present invention, there is provided a composition for oral consumption comprising genistein and docosahexaenoic acid (DHA) and less than 1% by weight of soy protein, wherein the weight ratio of genistein to DHA is in the range of from 1:100 to 1:1, the composition comprises genistein in an amount of from 0.0001% to 0.1% by weight, which comprises less than 0.01% by weight of a food grade phospholipid emulsifier. Preferably the genistein and DHA exhibit an anti-ageing effect on skin.

The invention involves the finding of a synergistic effect between DHA and genistein. The levels of DHA and genistein in the compositions of the invention are preferably selected so as to achieve a synergistic effect.

The genistein may be in glycosylated or non-glycosylated form, or a mixture of these two forms. Reference to genistein throughout this specification means the glycosylated or non-glycosylated forms, or mixtures of the two forms, unless specifically stated otherwise. Amounts of genistein are calculated based on non-glycosylated form (i.e., as if any glycosylated genistein were non-glycosylated). The genistein is preferably present in the composition of the invention as a component of a natural product or an extract or concentrate thereof. Preferably, the natural product is soy or red clover, more preferably soy.

The genistein, when it is from soy, is preferably purified at least to some extent by removal of soy protein. Therefore, compositions of the invention preferably contain less than 1% by weight of soy protein, more preferably less than 0.5% by weight of soy protein, even more preferably less than 0.1% by weight of soy protein, such as less than 0.01% or less than 0.001% or less than 0.0001% by weight. The composition of the invention may be free of soy protein or substantially free of soy protein.

The weight ratio of genistein to DHA in the composition of the invention is in the range of from 1:200 to 2:1, preferably from 1:100 to 1:1, more preferably in the range of from 1:80 to 1:5, even more preferably from 1:60 to 1:10, preferably from 1:50 to 1:15, such as from 1:40 to 1:20, for example 1:30 to 1:20.

The composition of the invention comprises genistein in an amount of from 0.0001% to 0.1% by weight, preferably from 0.001% to 0.05% by weight, more preferably from 0.005% to 0.04% by weight, even more preferably from 0.005% to 0.025% by weight, most preferably from 0.01% to 0.025% by weight.

It is preferred that the genistein is present in the form of soy isoflavones. Therefore, it is preferred that the composition comprises from 0.01 to 0.5 wt% soy isoflavones. This is equivalent to from 10 to 500 mg/100g. Preferably the product contains from 0.01 to 0.3 wt% soy isoflavones.

The compositions of the invention comprise DHA. The DHA is preferably present in the form of a fish oil or is from a microbial source. The DHA may be in the form of a free acid, a C1 to C6 alkyl ester, a glyceride (including mono- di- and triglycerides) or mixtures thereof. Preferably, the DHA is in the form of a glyceride (e.g., a triglyceride). Reference herein to DHA means the free acid or alkyl esters or glycerides or mixtures thereof.

DHA is an ω-3, polyunsaturated, 22-carbon fatty acid. It is also present in abundance in certain fish (such as tuna and bluefish) and marine animal oils.

Typically, the amount of DHA in the compositions of the invention ranges from 0.001% to 4% by weight of the composition. More preferred amounts are from 0.01% to 5% by weight, such as from 0.1% to 1% by weight or from 0.1 to 0.5% by weight.

In one alternative embodiment, the composition may comprise less than 0.2% by weight oil comprising DHA. In another alternative embodiment, the composition may comprise more than 5% by weight oil comprising DHA.

The DHA may be present together with EPA.

Eicosapentaenoic acid (EPA) is one of several ω-3 fatty acids used by the body. Increased intake of EPA has been shown to be beneficial in coronary heart disease, high blood pressure, and inflammatory disorders such as rheumatoid arthritis.

Eicosapentaenoic acid (EPA) and docosahexaenoic acid (DHA) come from cold water fish such as wild salmon (not farm raised), mackerel, sardines, herring and other northern marine animals. Fish can make EPA and DHA from the ω3 essential fatty acid, alpha-linolenic acid (LNA), but get much of their EPA and DHA from brown and red algae which manufacture EPA and DHA from carbohydrates - sugar, starch, cellulose, etc.

More recently, brown and red algae have begun to be grown commercially for EPA and DHA. These make 10 to 14% of long-chain ω3s (on dry weight basis) and can be used as food sources of EPA and DHA-containing triglycerides.

Compositions of the invention are oral compositions, i.e. they are adopted for oral consumption. As such, the compositions are edible and non-toxic.

The composition of the invention is edible and is preferably water based, i.e. comprises at least 50wt% water, preferably at least 60wt% or even at least 70wt% water. It may be either liquid or frozen. The product thus has the sensation of being a regular water-based product and can be consumed on a regular basis as part of a consumer's normal diet. For example, it could replace a fruit juice normally consumed at breakfast time.

The composition of the invention may preferably be packaged as a beverage, for example in a container such as a carton or a bottle of coated paper or cardboard, glass or plastic. The container preferably has a volume of from 10 to 500 ml, such as from 20 to 100 ml.

In another embodiment, the composition of the invention comprises less than 50% by weight water and/or is substantially free of preservatives and/or flavouring.

The composition preferably comprises one or more further components selected from antioxidants, flavouring agents, preservatives and stabilisers.

The composition of the invention preferably has a pH of from 3 to 5, such as from 3 to 4.

Preferably the composition has a viscosity of from 2 to 100 centipoise at a shear rate of 1s⁻¹ and at 25°C.

The composition of the invention may take any suitable form, including, for example, food products and nutritional supplements. Compositions for oral consumption which may be used according to the invention include beverages, bars and other liquid and solid forms such as tablets, pills, capsules and powders (which may contain crystalline material), as well as spreads, margarines, creams, sauces, dressings, mayonnaises, ice creams, fillings, confectionaries and cereals.

Preferably, the compositions of the invention are in the form of a substantially homogeneous aqueous emulsion, suspension or dispersion.

The composition of the invention is preferably packaged as a beverage.

One or more antioxidants are preferably present in the compositions of the invention in order to prevent or slow down the natural oxidative degradation of the DHA. Rancid fish oil not only has an unpleasant taste but may even have negative health effects (Kubow S., "Toxicity of dietary lipid peroxidation products", Trends in Food Sciences & Technology, September, 67-71 (1990)).

Suitable antioxidants can be selected, although not exclusively, from the following list, either singularly or in combination: TBHQ, Ascorbyl esters (e.g. ascorbyl palmitate), ascorbic acid, Tocopherols, Rosemary Extract; fruit concentrates or extracts, black or green tea extract, Propyl Gallate, essential oils or oleoresins, butylated hydroxyanisole (BHA), butylated hydroxytoluene (BHT), citric acid or esters, co enzyme Q10, Tocotrienols, Chelators (e.g. EDTA), Carriers, polyphenols, phenolic compounds, flavonoids, oxygen scavengers.

Especially preferred antioxidants are vitamins C and E. Not only are these effective antioxidants but they also have been shown to give skin benefits when consumed.

An amount of antioxidant should be added sufficient to prevent the DHA from going rancid over a typical shelf-life of 6 months. Clearly the amount of antioxidant will depend on the type and activity of the antioxidant used. However, preferably the product has a weight ratio of antioxidant to oil of from 1:10 to 1:100 based on the antioxidant activity of vitamin C. For example, if an antioxidant with twice the activity of vitamin C was used, the ratio would be from 1:20 to 1:200.

For these purposes an antioxidant activity is as measured using an appropriate assay (e.g. Trolox equivalent antioxidant capacity).

The compositions of the invention preferably comprise a flavouring. Suitable flavouring agents may be natural or synthetic. Flavouring may be required to make the product more palatable for consumption.

It is preferred that the composition contains at least 0.01 wt% food-grade phospholipid emulsifier. Preferably, the emulsifier is present in an amount of from 0.05 to 3 wt%, more preferably from 0.1 to 1 wt%.

Phospholipid emulsifiers were found to be very suitable.

A food grade phospholipid emulsifier may be required in order to carry DHA in a oil-in-water emulsion. It is preferred that the phospholipid emulsifier is lecithin. Phospholipid emulsifiers are oil soluble, but the lecithin can be added to either phase prior to emulsification. Preferably it is added to the aqueous phase.

The product may also comprise from 0.0005 to 0.1 wt% carotenoids. This is equivalent to from 0.5 to 100 mg/100g. Preferably, the product contains from 0.002 to 0.04 wt% carotenoids. The carotenoids, being oil soluble, would be comprised predominantly within the oil phase. Highly preferred carotenoids are β-carotene, and lycopene. These carotenoids provide moderate protection from UV induced erythema, thought to be due to their antioxidant functionality including scavenging of reactive oxygen species.

The composition of the invention is typically consumed from one to four times daily (preferably once daily).

The composition may produce an anti-ageing effect on skin. By the term "anti-ageing", we mean that the skin may appear less wrinkled (i.e., there is an anti-wrinkling effect on wrinkles and/or fine lines) and may have one or more further benefits for the skin selected from: reduced dryness; increased firmness; increased elasticity; increased smoothness; less inflammation; clearer skin; fewer spots, pimples and blemishes (including acne); clearer skin; less sensitive skin; and generally healthier skin. Compositions of the invention may exhibit the anti-ageing effect by increasing collagen synthesis in the skin and compositions of the invention may be used to increase collagen synthesis (as part of, or separately from, the anti-ageing effect); preferably collagen synthesis is increased by at least 10%, more preferably at least 20% such as at least 25% (by weight preferably over a 14 week period, based on a control without added DHA and genistein). The skin may include the skin of the whole body, preferably the face, neck and/or hands. The skin may also include scalp skin with benefits for hair (including reduced ageing) and scalp itch or irritation. Conveniently, the benefit can be cosmetic.

The product of the present invention can be prepared from an aqueous phase and an oil phase. In general the water-soluble ingredients are put together in the aqueous phase and the oil-soluble ingredients in the oil phase. The exception is the emulsifier. It has been surprisingly found that the emulsifier, which is oil-soluble, gives a more stable emulsion when it is added to the aqueous phase.

The two phases are then blended together in conventional emulsifier equipment. The produced emulsion is shelf-stable and the oil does not go rancid for months.

The oil phase and aqueous phase are then blended together to form a homogenous stable emulsion.

In a preferred process the oil is on a powdered carrier material to assist emulsion formation.

The stable emulsion may then be packaged in a sealed container such as a metal, coated cardboard (e.g. tetra Pak) or plastic container. The container is then preferably sealed so as to give no headspace or a gas filled (e.g. nitrogen or carbon dioxide) headspace. This assists still further in preventing the DHA or fish oil oxidising.

Alternatively the emulsion may be frozen and packaged and sold as a frozen consumer product.

In an alternative embodiment, the composition of the invention is contained in a capsule. Typically, the component of the composition may then be in a more concentrated form. The capsule may be made of any suitable material well known in the art such as gelatin. The capsule is adapted to be swallowed by the consumer and typically one or two capsules will be taken from one to four times per day. Each capsule preferably comprises from 10 to 4000 mg of polyunsaturated fatty acid (more preferably from 10 to 3000 mg or 20 to 2000 mg or 20 to 1000 mg, even more preferably from 50 mg to 500 mg) and from 10 to 500 mg of soy isoflavones, or a mixture thereof (preferably from 20 mg to 500 mg, such as from 30 mg to 250 mg or from 40 mg to 150 mg).

In a further contemplated embodiment, when the composition of the invention is provided as a tablet, liquid, capsule or powder, the active ingredients in the invention may be distributed across different capsules, tablets, pills or powders, so that for instance the user may be provided with one tablet, capsule, pill or powder containing the DHA, and another tablet, capsule, pill or powder containing the genistein. In such an instance, the user is also ideally provided with instructions to take the liquids, capsules, pills or powders according to a dosage regime, which may entail taking them in the same dose, i.e. at the same time or in quick succession.

In yet a further embodiment, the composition of the invention may be included as one component of a complex food product. For instance, the composition may be present in a solid or gelatinous form as a filling or layer within a bar, or a similar product. The composition of the invention may therefore be included in a wide range of everyday foodstuffs, for instance in "health food" bars which could be eaten as an alternative to other food snacks.

In a further embodiment, the kit of parts may additionally or alternatively comprise a number of separately packaged beverages, wherein the consumer will typically drink one beverage per day. Also within such a kit of parts may be found a composition for topical application to the skin, wherein the quantity of topical composition supplied is intended to provide for use by the consumer for the same number of days as beverages supplied.

The beverage may also be sold in a single package, from which it is intended the consumer drink a measured amount per day.

The topical composition may be provided in amounts which will allow for continued use of the topical composition beyond the time when the beverage has been used, similarly, more beverage may be provided so that this may continue to be consumed beyond the time when the supply of topical composition has run out.

Further, the kit of parts may comprise a packaged product in which different supplements are packaged together, optionally with use instructions, in order to achieve the benefits of the invention. For instance, the kit may comprise capsules, tablets pills and/or powders including separately or in combination salicylic acid, a C1 to C6 alkyl ester thereof and a salt thereof and a polyunsaturated fatty acid. It is possible that this kit would also include a topical composition.

A kit of parts would typically offer a product which could be used for from about 1 week to about 3 months, often about 2 weeks to about 2 months, most preferably for about 1 month.

The following non-limiting examples illustrate the invention and do not limit its scope in any way. In the examples and throughout this specification, all percentages, parts and ratios are by weight unless indicated otherwise.

### EXAMPLES

### Determination of Increase in Collagen Synthesis

### Outline of Experimental Approach

A biochemical assay and protein extraction method was developed to determine changes in new collagen synthesis in the skin.
a. Skin biopsies were taken at baseline (T1) and end (T15) of the intervention period.
b. At each time point, two 3mm punch biopsies (4mm depth) were taken, placed in a cryotube container and immediately snap frozen in liquid nitrogen.
c. These biopsies were then stored at -80°C.

### Materials and Methods

### Preparation of Cell lysate

All punch biopsies were placed in a dounce homogeniser with 1 ml cell lysis buffer and ground up completely (so as no significant lumps of skin or extracellular matrix remained). The lysis buffer contained 1% NP-40, 0.1% sodium deoxycholate, 0.1% SDS, 6 mM sodium chloride and 0.05M Tris at pH 7.6. Protease inhibitor cocktail (1000X; Sigma P8340) was added prior to use at a level of 10 µl per ml of lysis buffer. Following complete homogenisation of the tissue, unwanted cell debris was removed by centrifugation for 20 minutes at 20,000g at 4°C. The clarified cell lysate was frozen at -80°C until needed.

### Total Protein Assay (Pierce)

The total protein concentration of each cell lysate was measured using the Pierce BCA protein assay kit. A set of eight standard solutions ranging from 0 to 1200 µg/ml protein was prepared from the supplied 2 mg/ml BSA stock solution. 10 µl of standard or cell lysate was added to duplicate wells of a flat-bottomed, 96-well microtitre plate. The reagent solution was prepared according to the kit instructions from 50 parts reagent A and 1 part reagent B. 200 µl of the final reagent was added to each well of the microtitre plate. The plate was mixed, covered and incubated at 37°C for 30 minutes and absorbance read at 562 nm. A protein standard curve was constructed and used to determine the protein concentration of each cell lysate.

### Procollagen I C-Peptide EIA KIT (Takara Bio Inc.)

Collagen I is synthesised as a precursor molecule, Procollagen I. The amount of free propeptide therefore, reflects stoichiometrically, the amount of collagen I synthesised. The Procollagen Type I C-peptide Enzyme Immunoassay (EIA) kit allows for the quantitative determination of Procollagen Type I C-peptide (PIP).

Eight PIP standards were prepared in sample diluent at concentrations ranging from 0 to 640 ng/ml. 100 µl of antibody-Peroxidase conjugate solution and 20 µl of cell lysate (1 µg protein) or standard was added to duplicate wells. The plate was sealed and incubated at 37°C for 3 hours before being washed four times with 400 µl of PBS. Each well then received 100 µl of substrate solution and the plate incubated at room temperature, on the benchtop, for 15 minutes. After this period, 100 µl stop solution was added to each well and absorbance measured at 450nm with a plate reader.

A standard curve was plotted of mean absorbance versus PIP concentration and the line of best fit calculated by regression analysis. The unknown concentration of PIP in all the samples was estimated from this.

### Measurement of skin hydration

Various methods for determining the hydration state of the stratum corneum have been summarized by Fluhr et al., Skin Res Technol 1999; 5:161-170. Briefly, the Corneometer (Courage & Khazaka) measures skin hydration through detection of epidermal capacitance. The probe is made of two finger-type metal plates close to each other, with a measurement depth of approximately 30 mm. The instrument determines the humidity level of the most external cutaneous layers of the stratum corneum. The action principle of the Corneometer^{®} is based on the modification of the electrical capacities of the detector which is designed in the form of a condenser. The surface of the measurement head, in contact with the skin, modifies its electrical capacity according to the humidity level of the skin. An increase in the value measured by the corneometer is indicative of improved skin hydration.

### Measurement of trans epidermal water loss (TEWL)

An analysis of methods to measure TEWL has been performed by Wilson & Maibach, (1989) Transepidermal water loss, A review, In: Cutaneous Investigation in Health and Disease, Non-invasive Methods and Instrumentation (Leveque, J. L., ed.), pp. 113-130, Dekker, New York, NY. The cutaneous barrier acts as a regulator in skin water balance. When this is damaged, the water exchange regulation system becomes destabilised. This means that water migrates more easily to the outside environment, increasing Transepidermal Water Loss. The effectiveness of the cutaneous barrier decreases with age. However, if the condition of the cutaneous barrier improves, water loss decreases as the water exchange regulation mechanism recovers its balance. TransEpidermal Water Loss measurements can be performed with a Servomed "Evaporimeter" EP-3^{®}. A probe made up of two captors is traversed by a flow of water vapor. The difference of the partial pressure is measured between the two captors. This value corresponds to the evaporation speed of a volatile substance (in this case, water). A reduction in TEWL is indicative of improved skin barrier properties

### Measurement of skin elasticity & firmness

Measurements for skin elasticity and firmness are made with a cutometer and described in Escoffier et al, J Invest Dermatol, 93(3):353-7. The measurement is done with an instrument which, using the vacuum principle, sucks up a defined area of skin surface and records it optically. Analysis of the recorded measurement curves makes it possible to determine the elastic and plastic characteristics of the skin. Young skin shows a high degree of elasticity and loses shape only gradually while regaining its original state after the end of the suction procedure. Skin which is young, healthy, supple and adequately moist will have a higher elasticity than an aged dry, rough skin. The cutometer therefore gives a set of measurements which allows us to quantify elastic characteristics. The technique consists of skin aspiration by a measurement probe. The skin is sucked into the orifice of the probe by negative pressure created within the device. The depth to which the skin penetrates into the probe is measured by a non-contact optical measurement system. This system consists of a light source and light receptor, as well as two prisms facing each other, which project the light from transmitter to receptor. Light intensity varies with penetration depth of the skin. The resistance of the skin to be sucked up gives an indication of the *firmness* of the skin and the ability to return to its original position gives an indication of the *elasticity* of the skin. A curve is displayed at the end of each measurement which allows several calculations to be made corresponding to skin mechanical properties.

### Analysis of fine lines, wrinkles & skin smoothness

Skin roughness and wrinkling can be assessed using replicas and skin profilometry as described by Cook, J Soc Cosmet Chem, 1980; 31:339-359. A silicon rubber material such as Silflo is prepared and applied to the test area. Once set it is removed and analysed using optical profilometry. With this measurement method, a parallel stripe pattern is projected onto the skin surface and depicted on the CCD chip of a camera. The 3D measurement effect is achieved by the fact that minute evaluation differences on the skin surface deflect the parallel projection stripes and that these deflections constitute a qualitative and quantitative measurement of the skin profile. The skin profiles are recorded by the CCD camera, digitised, and transferred to the measurement and evaluation computer for qualitative evaluation.

### Example 1 - Anti-inflammatory synergistic effect between genistein and DHA in human umbilical vein endothelial cells (Huvec's).

### Outline of Experimental Approach

An *in vitro* model has been developed to investigate the impact of cytokine stress on the inflammatory status of endothelial cells.
a. Cells are grown in 6-well (9.5cm²) plates.
b. The cells are treated with 0.1ng/ml Interleukin 1-beta (IL1-beta).
c. Tissue culture supernatant and cell pellets were harvested at 24 hours (t24) post-IL1-beta treatment.
d. All tissue culture supernatant was assayed for Lactate Dehydrogenase (LDH), as a measure of cytotoxicity, and Interleukin 6 (IL6) synthesis.
e. All cells were counted (Beckman Coulter Counter) and pelleted and cell lysate assayed for Intra Cellular Adhesion Molecule 1 expression (ICAM-1).

### Materials and Methods

### Culture of Endothelial Cells

Huvec cells (Human umbilical vein endothelial cells, TCS Biologicals) were cultured and passaged in EGM-2 (Endothelial growth medium, Biowhittaker) supplemented with heparin, VEGF (vascular endothelial growth factor), gentamicin sulphate, ascorbic acid, HEGF (Human endothelial growth factor), hydrocortisone, HFGF-B (Human fibroblast growth factor B), R3-IGF-1 (long R insulin-like growth factor 1) and FBS (foetal bovine serum).

Cells were routinely plated out in 6-well tissue culture dishes, at a seeding density of about 5000 cells/cm² in 2ml complete medium/well, 24 hours before starting the experiment, and incubated at 37°C in 5% CO₂.

### Addition of Test Solutions

Test solutions were prepared in EGM-2 containing all supplements except hydrocortisone. Endothelial cells were treated for 24 hours with 0.1 ng/ml ILlbeta.

### Harvesting Samples and Cell Number

Any change in cell morphology was noted before the cells were harvested. Both the tissue culture supernatant and the endothelial cells were harvested after addition of recombinant IL1-beta (t24). All tissue culture supernatants were stored at - 20°C.

1 ml of trypsin/EDTA solution (Invitrogen 25300-054) was added to each well, and the plate incubated at 37°C until the cells detached. 50µl of this cell suspension was added to 9.95mls of Isoton II (Beckman Coulter) in an accuvette and 0.5ml of this suspension was counted twice in a Coulter Particle Counter Z1 with 140µm aperture.

The remaining 950 µl of original cell suspension was centrifuged at 13000 rpm in a microcentrifuge for 10 minutes. The supernatant was discarded and the cell pellet washed with 500µl of Dulbecco's PBS and centrifuged as before. The supernatant was discarded as before and cell pellet stored at -20°C prior to cell lysis. The number of cells per pellet was estimated from the Coulter Counter data.

### Cytotoxicity Assay (Promega)

All tissue culture supernatant was examined for cytotoxicity using the Promega CytoTox 96 non-radioactive cytotoxicity assay. This assay quantitatively measures lactate dehydrogenase (LDH) released upon cell lysis and is a good indication of cell viability. 50 µl of tissue culture supernatant or control medium was added to duplicate wells of a 96-well microtitre plate. 50 µl of CytoTox reagent was added and mixed well. The plate was incubated in the dark, at room temperature, for 30 minutes. After this time 50 µl of stop solution was added to each well and the absorbance of the plate was read at 492nm. Any test samples giving an absorbance value of more than double that of the control medium was considered to be cytotoxic. No results have been included from samples that showed any signs of cytotoxicity.

### Preparation of Cell lysate

All cell pellets were lysed on ice for 30 minutes in 1 ml cell lysis buffer per 2.5 x 10⁶ cells. The lysis buffer contained 1% NP-40, 0.1% sodium deoxycholate, 0.1% SDS, 6 mM sodium chloride and 0.05M Tris at pH 7.6. Protease inhibitor cocktail (1000X; Sigma P8340) was added prior to use at a level of 10 µl per ml of lysis buffer. The partially lysed cell pellets were completely homogenised with a pellet pestle and unwanted cell debris removed by centrifugation for 20 minutes at 20,000g at 4°C. The clarified cell lysate was frozen at -80°C until needed.

### Total Protein Assay (Pierce)

The total protein concentration of each cell lysate was measured using the Pierce BCA protein assay kit. A set of eight standard solutions ranging from 0 to 1200 pg/ml protein was prepared from the supplied 2 mg/ml BSA stock solution. 10 µl of standard or cell lysate was added to duplicate wells of a flat-bottomed, 96-well microtitre plate. The reagent solution was prepared according to the kit instructions from 50 parts reagent A and 1 part reagent B. 200 µl of the final reagent was added to each well of the microtitre plate. The plate was mixed, covered and incubated at 37°C for 30 minutes and absorbance read at 562 nm. A protein standard curve was constructed and used to determine the protein concentration of each cell lysate.

### ICAM-1 ELISA (R&D Systems)

ICAM-1 protein in each cell lysate was estimated using the Human sICAM-1 DuoSet ELISA kit (R&D Systems DY720) according to the manufacturer's instructions.

The capture antibody was diluted to a final concentration of 4 µg/ml in PBS and 100 µl was used to coat each well of a 96-well microtitre plate overnight at room temperature. The plate was then washed three times with wash buffer (0.05% Tween 20 in PBS). Each well received 300 µl of blocking buffer (1% BSA, 5% sucrose and 0.05% sodium azide in PBS), and the plate was incubated at room temperature for 1 hour before being washed as before. Each cell lysate was then diluted 1/200 in reagent diluent (1% BSA in PBS) and 100 µl added to duplicate wells of the antibody coated plate. Eight ICAM-1 standards were prepared in reagent diluent, at concentrations ranging from 0 to 1000 pg/ml, and duplicate 100 µl standards were added to the appropriate wells on the plate. A separate set of standards was routinely used for each plate. The plate was incubated at room temperature for 2 hours before being washed again. 100 µl of detection antibody, diluted to a final concentration of 100 ng/ml, was added to each well and the plate incubated at room temperature for 2 hours. The plate was washed as before. Each well received 100 µl of streptavidin-HRP conjugate diluted 1/200 in reagent diluent and the plate incubated at room temperature, in the dark, for 20 minutes. The plate was washed for the final time and 100 µl of substrate solution (1:1 mixture of colour reagent A and colour reagent B, R&D Systems DY999) was added to each well. After 20 minutes incubation, at room temperature in the dark, the colour development was stopped by the addition of 50 µl of 2N sulphuric acid. The absorbance of the plates was measured at 450nm with the correction wavelength set at 570nm.

A standard curve was plotted of mean absorbance versus ICAM-1 concentration and the line of best fit calculated by regression analysis. The unknown concentration of ICAM-1 in the samples was calculated from this, taking the lysate dilution factor into account.

To normalise for differences in cell number and total protein concentration, the final result was expressed as ng ICAM-1 per mg of total protein.

### Interleukin-6 ELISA (R&D Systems)

The IL-6 protein concentration of each tissue culture supernatant was assayed using the QuantiGlo Q6000 Human IL-6 assay (R&D Systems) according to the manufacturer's instructions.

Six IL-6 standards were prepared in calibrator diluent at concentrations ranging from 0 to 3000 pg/ml. 50 µl of assay diluent and 150 µl of tissue culture supernatant or standard was added to duplicate wells. The plate was incubated at room temperature for 2 hours on a horizontal orbital plate shaker before being washed four times with wash buffer. 200 µl of IL-6 conjugate was added to each well and the plate incubated at room temperature for 3 hours on a horizontal orbital plate shaker (about 500rpm). The plate was washed as before. Each well received 200 µl of substrate solution and the plate incubated at room temperature, on the benchtop, for 40 minutes. The relative light unit (RLU) of each well was determined using a luminometer set with 1 minute lag time, 1 second/well read time, summation mode and automatic gain on.

A standard curve was plotted of mean RLU versus IL-6 concentration and the line of best fit calculated by regression analysis. The unknown concentration of IL-6 protein in all the samples was estimated from this.

### Prostaglandin E2 High Sensitivity ELISA (R&D Systems)

The PGE2 protein concentration of each tissue culture lysate was assayed using the DE2100 Human PGE2 assay (R&D Systems) according to the manufacturer's instructions.

Eight PGE2 standards were prepared in calibrator diluent at concentrations ranging from 0 to 1000 pg/ml. 150 µl of assay diluent and 50 µl of tissue culture lysate or standard was added to duplicate wells. 50µl of PGE2 HS antibody solution was added to each well and incubated for 18-24, hours at 2-8 °C. The plate was then washed four times with wash buffer. 200 µl of pNPP substrate was added to each well and the plate incubated at room temperature for 1 hour at 37°C. 50ul of stop solution was then added to each well. The optical density of each well was determined using a microplate reader set to 405nM with wavelength correction set between 570nM and 590nM.

A standard curve was plotted of mean RLU versus PGE2 concentration and the line of best fit calculated by regression analysis. The unknown concentration of PGE2 protein in all the samples was estimated from this.

Genistein and DHA were obtained from Sigma Aldrich.

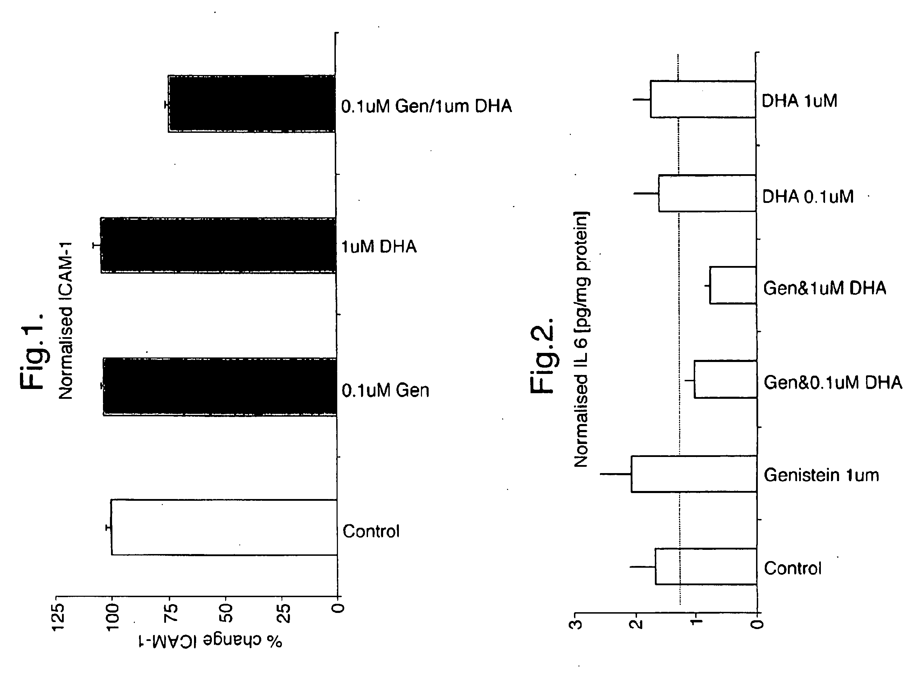

Figure 1 shows the synergy between genistein and DHA in human umbilical vein endothelial cells in terms of the changes in ICAM-1.

### Example 2 - Anti-inflammatory synergistic effect between genistein and DHA in human primary dermal fibroblasts.

### Outline of Experimental Approach

An *in vitro* model has been developed to investigate the impact of oxidative stress on the inflammatory status of dermal fibroblast cells.
a. Cells are grown in 6-well (9.5cm²) plates.
b. The cells are oxidatively stressed with 1 µM Phorbol Myristate Acetate (PMA).
c. Tissue culture supernatant and cell pellets were harvested at 24 hours (t24) post-PMA treatment.
d. All tissue culture supernatant was assayed for Lactate Dehydrogenase (LDH), as a measure of cytotoxicity and Interleukin-6 synthesis.
e. All cells were counted (Beckman Coulter Counter) and pelleted and cell lysate assayed for Procollagen-1 (PC-1) and Prostaglandin E2 (PGE2) expression.

### Materials and Methods

### Culture of Dermal Cells

Primary human dermal fibroblast cells were cultured and passaged in DMEM (Gibco) supplemented with 10% FBS (foetal bovine serum). Cells were routinely plated out in 6-well tissue culture dishes, at a seeding density of about 5000. cells/cm² in 2ml complete medium/well for 24 hours, and incubated at 37°C in 5% CO₂. Media was removed and cells grown in DMEM & 1% FBS, 24 hours prior to treatments.

### Addition of Test Solutions

Test solutions were prepared in DMEM containing low serum (1% FBS). Dermal fibroblasts were oxidatively stressed for 24 hours with 1 µM PMA (Sigma P8139).

### Procollagen I C-Peptide EIA KIT (Takara Bio Inc.)

Collagen I is synthesised as a precursor molecule, Procollagen I. The amount of free propeptide therefore, reflects stoichiometrically, the amount of collagen I synthesised. The Procollagen Type I C-peptide Enzyme Immunoassay (EIA) kit allows for the quantitative determination of Procollagen Type I C-peptide (PIP).

Eight PIP standards were prepared in sample diluent at concentrations ranging from 0 to 640 ng/ml. 100 µl of antibody-Peroxidase conjugate solution and 20 µl of cell lysate (1 µg protein) or standard was added to duplicate wells. The plate was sealed and incubated at 37°C for 3 hours before being washed four times with 400 µl of PBS. Each well then received 100 µl of substrate solution and the plate incubated at room temperature, on the benchtop, for 15 minutes. After this period, 100 µl stop solution was added to each well and absorbance measured at 450nm with a plate reader.

A standard curve was plotted of mean absorbance versus PIP concentration and the line of best fit calculated by regression analysis. The unknown concentration of PIP in all the samples was estimated from this.

Figure 2 shows the synergy between genistein and DHA in human primary dermal fibroblasts in terms of changes in IL6.

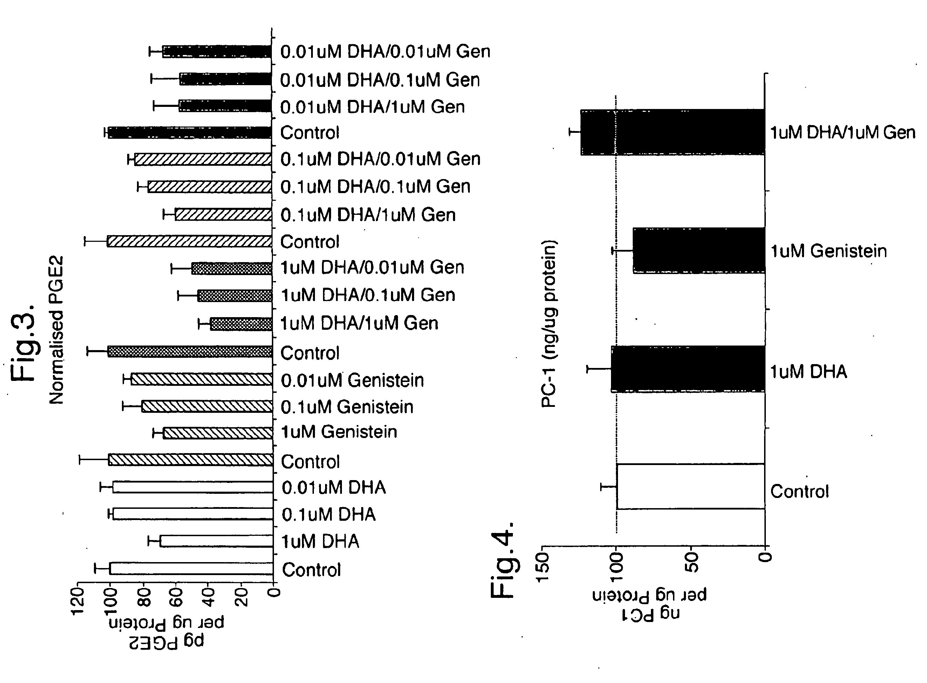

Figure 3 shows the synergy between genistein and DHA in human primary dermal fibroblasts in terms of changes in PGE2.

Figure 4 shows the synergistic stimulation of procollagen 1 in human dermal fibroblast cells treated with DHA and genistein.

### Example 3 - Composition of the Invention

The following is an example of a composition of the invention.

| **Ingredient** | **Weight %** |
|---|---|
| DHA | 0.40 |
| Genistein | 0.017 |
| Vitamin C | 0.17 |
| Vitamin E | 0.25 |
| Lycopene | 0.005 |
| Beta-carotene | 0.002 |
| Citric acid | 0.18 |
| Flavouring, sweetener, thickener, emulsifier | q.v. |
| Water | To 100% |

The composition can be prepared by adding the components to water and homogenising the mixture.

## Claims

1. Composition for oral consumption comprising genistein and docosahexaenoic acid (DHA) and less than 1% by weight of soy protein, wherein the weight ratio of genistein to DHA is in the range of from 1:100 to 1:1, the composition comprises genistein in an amount of from 0.0001% to 0.1% by weight, which comprises less than 0.01% by weight of a food grade phospholipid emulsifier.

2. Composition according to claim 1, which is in the form of a substantially homogeneous aqueous emulsion, suspension or dispersion.

3. Composition according to claim 1 or claim 2, wherein the genistein and DHA exhibit an anti-aging effect on the skin.

4. Composition as claimed in any one of the preceding claims, wherein the genistein is present as a component of a natural product or an extract or concentrate thereof.

5. Composition as claimed in Claim 4, wherein the natural product is soy.

6. Composition as claimed in any one of the preceding claims, wherein the DHA is present in the form of a fish oil or is from a microbial source.

7. Composition as claimed in any one of the preceding claims, which comprises one or more further components selected from antioxidants, flavouring agents, preservatives and stabilisers.

8. Composition as claimed in any one of preceding claims, which is packaged as a beverage or a bar, preferably a beverage.

9. Composition as claimed in any one of the preceding claims, which has a pH of from 3 to 5.

10. Composition as claimed in any one of the preceding claims, which comprises less than 50% by weight water and/or is substantially free of preservatives and/or flavouring.

11. Composition as claimed in any one of the preceding claims, which comprises less than 0.2% by weight oil comprising DHA.

12. Composition as claimed in any one of Claims 1 to 11, which comprises more than 5% by weight oil comprising DHA.

13. Composition as claimed in any one of the preceding claims, wherein the composition is in the form of a food product or supplement.

14. Composition as claimed in any one of Claims 1 to 12, wherein the composition is in the form of a tablet, pill, capsule or powder.

## Patentansprüche

1. Zusammensetzung zum oralen Verzehr, die Genistein und Docosahexaensäure (DHA) und weniger als 1 Gew.-% Sojaprotein umfasst, wobei das Gewichtsverhältnis von Genistein zu DHA im Bereich von 1:100 bis 1:1 liegt, wobei die Zusammensetzung Genistein in einer Menge von 0,0001 Gew.-% bis 0,1 Gew.-% und weniger als 0,01 Gew.-% eines Phospholipid-Emulgators mit Lebensmittelqualität umfasst.

2. Zusammensetzung gemäß Anspruch 1, welche in Form einer im Wesentlichen homogenen wässrigen Emulsion, Suspension oder Dispersion vorliegt.

3. Zusammensetzung gemäß Anspruch 1 oder Anspruch 2, wobei Genistein und DHA eine Anti-Aging-Wirkung auf die Haut zeigen.

4. Zusammensetzung, wie sie in einem der vorangehenden Ansprüche beansprucht wird, wobei das Genistein als eine Komponente eines natürlichen Produkts oder eines Extrakts oder Konzentrats davon vorliegt.

5. Zusammensetzung, wie sie in Anspruch 4 beansprucht wird, wobei das natürliche Produkt Soja ist.

6. Zusammensetzung, wie sie in einem der vorangehenden Ansprüche beansprucht wird, wobei DHA in Form eines Fischöls vorliegt oder aus einer mikrobiellen Quelle ist.

7. Zusammensetzung, wie sie in einem der vorangehenden Ansprüche beansprucht wird, die eine oder mehrere weitere Komponente(n), ausgewählt aus Antioxidanzien, Aromamitteln, Konservierungsmitteln und Stabilisatoren, umfasst.

8. Zusammensetzung, wie sie in einem der vorangehenden Ansprüche beansprucht wird, die als Getränk oder Riegel, vorzugsweise als Getränk, verpackt ist.

9. Zusammensetzung, wie sie in einem der vorangehenden Ansprüche beansprucht wird, die einen pH von 3 bis 5 hat.

10. Zusammensetzung, wie sie in einem der vorangehenden Ansprüche beansprucht wird, die weniger als 50 Gew.-% Wasser umfasst und/oder im Wesentlichen frei von Konservierungsmitteln und/oder Aromamittel ist.

11. Zusammensetzung, wie sie in einem der vorangehenden Ansprüche beansprucht wird, die weniger als 0,2 Gew.-% Öl, das DHA umfasst, umfasst.

12. Zusammensetzung, wie sie in einem der Ansprüche 1 bis 11 beansprucht wird, die mehr als 5 Gew.-% Öl, das DHA umfasst, umfasst.

13. Zusammensetzung, wie sie in einem der vorangehenden Ansprüche beansprucht wird, wobei die Zusammensetzung in Form eines Nahrungsmittelprodukts oder eines Nahrungsergänzungsmittels vorliegt.

14. Zusammensetzung, wie sie in einem der Ansprüche 1 bis 12 beansprucht wird, wobei die Zusammensetzung in der Form einer Tablette, Pille, Kapsel oder eines Pulvers vorliegt.

## Revendications

1. Composition pour consommation orale, comprenant de la génistéine et de l'acide docosahexaènoïque (DHA) et moins de 1 % en poids de protéine de soja, dans laquelle le rapport pondéral entre la génistéine et le DHA est situé dans la plage allant de 1 : 100 à 1 : 1, la composition comprend de la génistéine en une quantité de 0,0001 % à 0,1 % en poids, qui comprend moins de 0,01 % en poids d'un émulsifiant à base de phospholipide de qualité alimentaire.

2. Composition selon la revendication 1, qui est sous la forme d'une émulsion, d'une suspension ou d'une dispersion aqueuse sensiblement homogène.

3. Composition selon la revendication 1 ou la revendication 2, dans laquelle la génistéine et le DHA présentent un effet antivieillissement sur la peau.

4. Composition selon l'une quelconque des revendications précédentes, dans laquelle la génistéine est présente en tant que composant d'un produit naturel ou de l'un de ses extraits ou concentrés.

5. Composition selon la revendication 4, dans laquelle le produit naturel est le soja.

6. Composition selon l'une quelconque des revendications précédentes, dans laquelle le DHA est présent sous la forme d'une huile de poisson ou provient d'une source microbienne.

7. Composition selon l'une quelconque des revendications précédentes, qui comprend un ou plusieurs composants supplémentaires choisis parmi les antioxydants, les agents aromatisants, les conservateurs et les agents de stabilisation.

8. Composition selon l'une quelconque des revendications précédentes, qui est conditionnée sous la forme d'une boisson ou d'une barre, de préférence d'une boisson.

9. Composition selon l'une quelconque des revendications précédentes, qui possède un pH de 3 à 5.

10. Composition selon l'une quelconque des revendications précédentes, qui comprend moins de 50 % en poids d'eau et/ou est sensiblement exempte de conservateurs et/ou d'aromatisants.

11. Composition selon l'une quelconque des revendications précédentes, qui comprend moins de 0,2 % en poids d'huile comprenant du DHA.

12. Composition selon l'une quelconque des revendications 1 à 11, qui comprend plus de 5 % en poids d'huile comprenant du DHA.

13. Composition selon l'une quelconque des revendications précédentes, dans laquelle la composition est sous la forme d'un produit ou d'un complément alimentaire.

14. Composition selon l'une quelconque des revendications 1 à 12, dans laquelle la composition est sous la forme d'un comprimé, d'une pilule, d'une capsule ou d'une poudre.
